# EUROPEAN PATENT SPECIFICATION

(11) **EP 1 094 780 B1**
(45) Date of publication and mention of the grant of the patent: **19.05.2004**
(21) Application number: 99933832.0
(22) Date of filing: 09.07.1999
(51) Int. Cl.: A61K 6/083, A61K 6/02

(54) **COMPOSITE VENEERED CAST GLASS-CERAMIC DENTAL CONSTRUCT**
MIT KOMPOSITMATERIAL VERKLEIDETE GEGOSSENE GLASKERAMIKZÄHNE
CONSTRUCTION DENTAIRE VITROCERAMIQUE MOULEE AVEC FACETTE EN MATERIAU COMPOSITE

(30) Priority: 10.07.1998 US 92484 P
(43) Date of publication of application: 02.05.2001
(73) Proprietor: New Age Biomaterials, Inc., Halifax, Nova Scotia B3H 2X1 (CA)
(72) Inventor: JONES, Derek, W., Halifax, Nova Scotia B3H 2X1 (CA); RIZKALLA, Amin, S., Halifax, Nova Scotia B3L 4T5 (CA); HALL, Gordon, C., Lower Sackville, Nova Scotia B4C 3G6 (CA)
(74) Representative: Hartz, Nikolai F., Dr.
(86) International application number: PCT/US1999/015548
(87) International publication number: WO 2000/002525

(56) References cited:
- EP-A- 0 022 655
- EP-A- 0 280 985
- US-A- 4 297 266
- US-A- 4 433 959
- US-A- 4 650 550
- US-A- 4 895 516
- US-A- 5 132 337
- US-A- 5 183 834

## Description

### FIELD OF THE INVENTION

The present invention relates to non-metallic dental products. More particularly, the invention relates to dental prosthetic devices formed from a composite veneered cast, sintered or hot pressed glass-ceramic material.

### BACKGROUND OF THE INVENTION

A major purpose of the dental profession has been to replace or correct damaged or deformed tooth structure or conditions by fabricating and installing dental constructs such as crowns, inlays, onlays, bridges (fixed partial dentures) and the like. These prostheses ideally should (1) be inert in the oral environment, (2) resist the forces of mastication, (3) be capable of assuming physiologically compatible anatomical configurations, and (4) exhibit aesthetic characteristics similar to those of natural teeth.

Present dental constructs are customarily composed of metal alloys, porcelain, amalgam, or acrylate polymers or combinations thereof, which do not completely meet the foregoing ideal requirements. Metal alloys and amalgam are undesirable in locations where aesthetics is a major consideration because they sharply differ from teeth in optical characteristics. Porcelain and acrylate polymers are either too brittle or too weak to resist masticatory forces in many locations. Composite structures, as in the case of an alloy substructure for strength and a porcelain superstructure for appearance are generally very technique sensitive and many times are very bulky. Therefore, prior dental constructs have been at best a compromise upon the four ideal requirements.

One drawback especially with the constructs employing metal alloys, is that these often release potentially toxic ions such as those from nickel in the base metal alloys. Another area with previous dental constructs have been deficient is in the ease of customizing shade and aesthetics of the material and matching the patient's existing dentition. Ceramic crowns have normally required greater involvement between the dental practitioner and the dental laboratory in order to adequately customize the shade and aesthetics. It has also been a problem that ceramic veneers are subject to fracture. A further problem encountered with the metal-ceramic veneered system is fracture of the ceramic veneer. Such a problem inevitably requires the expensive and complicated procedure of removal of the crown or bridge from the mouth in order for it to be repaired or remade by the dental laboratory. In this regard, it has been found that the greater the difference between the thermal expansion qualities of metal and that of the ceramic materials applied thereto with previous constructs, aggravate the problem of cracking or fissuring. Such problems become increasingly troublesome with the number of re-firing schedules of the ceramic-metal construct.

A need exists therefore, for a dental construct having ease of manufacture qualities. A need also exists for such a construct having improved aesthetic characteristics and biocompatability. The constructs should be easily customized for colour shading and shape at the chair-side. In order to avoid delamination of the veneering material, the substrate and the veneering material of a dental construct should ideally not be sensitive to slight mismatch between their thermal expansion characteristics. In addition the veneering material should not be subject to failure at very low strains.

### SUMMARY OF THE INVENTION

It is therefore, an object of the invention to provide a non-metallic dental construct.

It is another object of the invention to provide a dental construct in the nature of a dental prosthesis.

It is a further object of the invention to provide a less expensive construct that may be easily customized for colour, shade and shape by the dental practitioner at the chair-side.

It is a further object of the invention to provide a construct which may be repaired by the dental practitioner at the chair-side, without the necessity of removing the prosthesis from the mouth.

It is an additional object of the invention to provide a chemically inert non-metallic (ceramic) substrate coping which exhibits some degree of translucency.

It is still another object of the invention to provide a non-metallic dental construct having improved aesthetic characteristics.

It is still yet a further objective of the invention to provide a veneer for a dental construct having improved toughness characteristics.

It is still yet an additional object of the invention to provide a dental construct which avoids the necessity of close matching of the thermal expansion characteristics of the substrate and the veneering material.

In general, a dental construct according to the present invention comprises a glass-ceramic coping having a resin composite material in the form of a veneer bonded or otherwise affixed to at least a portion of the coping.

These and other objects of the present invention, which shall become apparent from the description in claims to follow, are accomplished by the invention hereinafter described and claimed.

### BRIEF DESCRIPTION OF THE DRAWINGS

Fig. 1 is a close-up, front elevational view of one dental construct according to the invention, namely a dental crown on a prepared tooth, the prepared portion of the tooth being shown in phantom lines.
Fig. 2 is an alternative embodiment of a dental construct according to the invention.

### PREFERRED EMBODIMENTS FOR CARRYING OUT THE INVENTION

There is provided according to the present invention, a dental construct having a glass-ceramic base or coping and a resin composite material in the form of a veneer bonded to the coping. The dental construct can be without limitation, a crown or bridge (fixed denture) restoration such as anterior or posterior crowns and bridges, Maryland-type bridges, onlays, inlays or any other type of dental restoration or appliance. The invention will be exemplified herein with respect to a crown 10 as depicted in the drawing, with the understanding that any dental construct is within the scope of the invention.

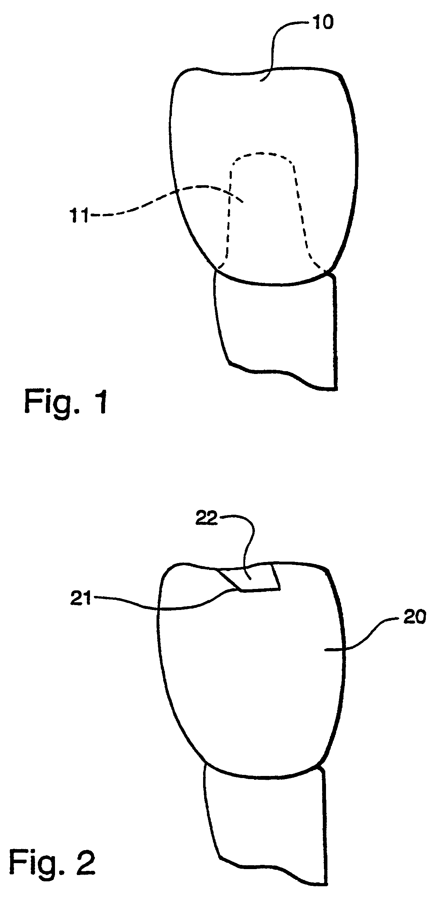

By way of example, Figure 1 depicts a prepared tooth 11 (shown partially in phantom lines) having a crown 10 affixed thereto. Crown 10 is affixed to prepared tooth 11 in any manner conventional with dental restorations, including cementatious and/or adhesive bonding.

The dental construct of the invention, such as crown 10, is preferably fabricated from a glass-ceramic material. Any method which is conventional in the art for preparing a glass-ceramic dental coping is within the scope of the invention. For example, the coping may be fabricated by being hot pressed, built-up by sintering frit using conventional methods, or by casting the glass-ceramic substructure using the lost wax casting method. An example of the fabrication of a glass-ceramic dental product is described in U.S. Pat. No. 4,431,420 which is hereby incorporated by reference for such disclosure.

A commercially available example of a glass-ceramic material is DICOR® brand glass-ceramic material available from Dentsply International Inc., York, Pennsylvania. It is known to be a tetrasilic-micaglass-ceramic K₂O-MgF₂-MgO-SiO₂.

Preferred glass-ceramic materials are Li₂O-CaO- Al₂O₃- SiO₂-X, Al(PO₃)₃-SiO₂-Li₂O-CaX and Li₂O-ZnO-P₂O₅-X, where X is a network modifier such as TiO₂, ZrO₂, La₂O₃, CeO₂, Y₂O₃, ZnO, MgO, BaO, PbO, Ta₂O₅, Li₂O, K₂O, CaF₂, MgF₂, AlF₃, BaF₂, and Na₂O. Such materials are preferably synthesized by wet chemical methods such as 1) Sol-gel polymerization of prehydrolyzed alkoxides (some soluble metal salts may be added becoming complexed into the gel). 2) The precipitation of precursors from suspensions by spray-drying, spray-freeze drying or freeze-drying. 3) Room temperature or elevated aqueous solution precipitation synthesis methods. 4) Hydrothermal synthesis in which the aqueous solutions or suspensions of precursor materials are heated at elevated temperatures and pressures. 5) Organic solution synthesis precipitation methods, and 6) Glycothermal synthesis in which the organic solutions or suspensions of precursor materials are heated at elevated temperatures and pressures. These methods allow homogeneous glasses and ceramics to be formed at temperatures well below the normal temperature required to sinter high density bodies of uniform microstructure.

Such compositions can be produced to allow them to be cast using the lost wax-casting process. A wax replica of the coping is produced sprued and invested in a refractory mold material, typically a silica refractory with a phosphate bonding agent is preferred. The burnout temperature for the mould is typically about 900°C, which should be heat soaked for at least 40 minutes prior to casting. The silica refractory is preferably in the form of amorphous silica and quartz rather than cristobalite. A lower compensation is required for the contraction of the glass on cooling following casting compared to a metal structure. The glass compositions identified above can be cast at temperatures from about 1,150 to about 1,450°C, and can be cerammed at temperatures between about 510 to 1200°C depending upon the composition. These materials posses good mechanical properties such as: modulus of elasticity values of 95-115 GPa compared to conventional dental porcelain which has a modulus of 65-75 GPa, fracture toughness values of between 1.2 and 1.8 K_{1C} (MPa m^{1/2}) and 'True Hardness' values of between 3.5 to 5.9 GPa.

A preferred method of casting the cerammable glass materials involves the use of an induction process to melt the glass or glass-ceramic starting material. A conducting insert is employed in the melting crucible to allow melting of the non-conducting glass to take place. A graphite or platinum insert is placed within the refractory crucible. The castings are removed from the investment mould by hand and lightly gritblasted with 25 µm alumina grains. Following the casting of the glass in the desired shape of the coping, such as the crown depicted as in Figure 1, the glass material is subjected to a controlled crystallization (ceramming) by a conventional heat treatment procedure. For example the cast construct (coping) is exposed to a temperature of 850°C for 30 minutes. The precise ceramming temperature for each glass composition has to be determined from the thermal analysis (DTA, curve). The heating can be performed in a gas fired or electric furnace with appropriate temperature control (±5°C).

According to the invention, the cerammed coping is then coated or at least partially coated, with a resin composite material in the form of a veneer, and contoured and shaped to simulate a natural tooth. Any composite material that is appropriate for use in the oral cavity is within the scope of the invention for use as the veneer coating. Preferred veneering materials include composites with dimethacrylate matrix resins. These matrix resins are generally mixtures of BIS-GMA and TEGDMA, in many commercial material, however urethane dimethacrylates and large oligomeric structures of BIS-GMA-urethanes may also be used.

Such composite materials having a blend of glass or ceramic particles dispersed in a polymerizable synthetic organic resin matrix. The polymer materials being blended together with finely divided inorganic (filler) reinforcing phase such as a calcium aluminosilicate, zirconium silicate, barium or strontium aluminosilicate glass or ceramics, preferably having a blending of large and small particles (0.04 to 10 µm) to obtain optimum packing density and mechanical properties. The so called hybrid systems may have either a bimodal or in some cases a trimodal blend of particle size. The trimodal systems lend themselves to a greater loading density. The size and distribution of the filler particles and the refractive index of filler and matrix resin should be oiptimized to give appropriate translucence for natural aesthetic results.

The filler particles of such composites preferably are surface treated to provide adhesion between the resin matrix and the glass or ceramic filler particles. Adhesion being achieved by using a silane (organo functional adhesion promoter) treatment. Most composite systems contain 2-5% of fumed silica to adjust viscosity and handling characteristics. This sub-micron silica is also generally treated with a silane coupling agent to reduce the uptake of water by the large surface area.

Such composite materials may use photopolymerizing systems activated by visible light in which a light sensitive absorber such as camphorquinone is used together with an aliphatic amine accelerator. However, chemically activated composite systems using the N,N-dimethyl-para-toluidine and benzoyl peroxide or similar system for chemical activation will be very appropriate, as will the heat curing systems since the veneering process is fabricated as an indirect system outside the mouth.

It is preferred, although not necessarily required, to prepare the coping to receive the veneer. For example, the coping may be etched by being physically contacted with a solution of hydrofluoric acid from 5 to 15 seconds (depending on the composition of the ceramic) to produce a micromechanical retentive surface. Cleaning of the ceramic surface with isopropyl alcohol, acetone or phosphoric acid is preferable, prior to silane treatment.

The etched and clean surface should preferably be treated with a coupling agent such as a hydrolyzed solution of aminopropoxyltriethoxy methacrylates silane or 3-(methoxyloxypropyl)-trimethoxysilane. A phosphoric acid solution can be added to the silane coupling agent to hydrolyse it prior to application on the ceramic surface. Alternatively a dilute solution of activated silane with ethyl alcohol can be used. The clean etched cerammed coping surface is then coated with the silane solution and dried for 30 minutes to one hour at 60°C.

The glass-ceramic coping will typically be from about 0.4 to about 1.00 mm in thickness. Greater thickness will be required in situations of higher masticatory stress (biting force). The overall combined thickness of coping and veneer will be dictated by function and aesthetics. However, typically the veneer would range from about 0.2 to as high as about 2.00 mm. The viscosity of the veneering resin (which may be as high as 100,000 cp or more) may be too high to allow good wetting of the surface of the silane treated ceramic coping substrate. The viscosity of the first coating of veneer can be reduced if necessary by use of low molecular weight monomer dimethacrylate diluents, such as ethylene glycol dimethacrylate (3.40 cp). In order to build up the veneer coating to the desired shape, thickness, contour and translucency/colour combination, a layering technique will be appropriate. Through subsequent applications the desired result can be obtained. In the case of photopolymerizing resin systems, curing can be accomplished at various stages of the build up. Natural tooth characteristics of translucency and colour can be incorporated during the seqential build up process.

It will be appreciated by one skilled in the art that a veneered structure according to the present invention has the strength, toughness and molulus of elasticity of a glass-ceramic structure with good marginal fit, combined with a tough composite coating which can be easily fabricated, with the option of adjustment of aesthetics and repair even when in place in the patient's mouth.

According to another aspect of the present invention, an inlay is prepared having a hollow cavity therein. As shown in Fig. 2, tooth 20 is prepared to receive an inlay 21. That is, most likely, tooth 20 will have had a damaged or decayed area (not shown) removed by conventional dental techniques. Inlay 21 having an open area 22 therein is formed according to any technique as was discussed above, and in the shape of the excavated area of tooth 20. Inlay 21 may be referred to as a "hollow box inlay". The cerammed, etched and silane treated hollow box inlay 21 is cemented or otherwise affixed to tooth 20 by conventional techniques. The relatively stiff ceramic supports the tooth 20 at the margins. The open area 22 of hollow box inlay 21 is then filled with a resin material such as a dimethacrylate as was discussed hereinabove. It will be appreciated that when worn, the resin material can be easily replaced while the margins will remain intact supported by the stronger and more rigid ceramic.

It will also be appreciated that an advantage of the present invention over previous dental constructs is the ability of the dental practitioner to customize the color (shade and physical aesthetics) of the veneering material. The veneer can also be easily repaired, changed or replaced with a subsequent veneer at the chair-side by the dental practitioner.

### GENERAL EXPERIMENTAL

In order to demonstrate the practice of the present invention, a number of examples of dental constructs have been prepared.

Several experimental glass/ceramic formulations have been successfully cast and cerammed in the form of constructs (copings) for dental crowns. This glass-ceramics had a general composition in the system SiO₂-Al₂O₃-La₂O₃-CeO₂-CaO-Li₂O. Examples of the glass-ceramic formulations are illustrated in Table 1.

**TABLE 1.**

| Examples of Compositions of Castable Glass-Ceramic Materials | | | | | | |
|---|---|---|---|---|---|---|
| Materials | %SiO₂ | %Al₂O₃ | %La₂O₃ | %CeO₂ | %CaO | %Li₂O |
| NAB/CG/7c | 44 | 16 | 23 | 6 | 5 | 6 |
| NAB/CG/10a | 59 | 12 | 13 | - | 8 | 8 |
| NAB/CG/10b | 49 | 12 | 23 | - | 8 | 8 |
| NAB/CG/L1 | 44 | 16 | 38 | - | 2 | - |
| NAB/CG/L3 | 36 | 16 | 46 | - | 2 | - |
| NAB/CG/L4a | 32 | 16 | 50 | - | - | 2 |
| NAB/CG/L5 | 30 | 15 | 53 | - | 2 | 7 |

The reduction to practice has been achieved by examples of these castings being veneered with a ceramic/resin composite material to produce crowns with acceptable aesthetics. The development of the idea of an induction melting casting technique for the glass proved to be an excellent procedure with first class results. Sections as thin as 200 µm have been successfully cast. Four of the different glass/ceramic formulations which have been cast have been found to have excellent mechanical properties which are much better than the commercial castable glass-ceramic materials currently available as indicated in Table 2. The highest fracture toughness and the second highest true hardness was exhibited by NAB/CG/7c which also had the lowest thermal expansion coefficient. A low thermal expansion coefficient is desirable since less compensation has to be made for the thermal shrinkage which occurs following cooling down after casting. The softening temperatures ranged from 863 to 1075°C for these materials. The thermal coefficient of expansion values for the examples shown in Table 2 ranged from 2.03 to 5.88 X 10⁻6/°C. All of the formulations were cerammable (capable of precipitating crystalline phases following heat treatment) which resulted in a significant increase in the values for mechanical properties such as fracture toughness and modulus of elasticity.

**TABLE 2 -**

| Examples of Properties for Castable Glass-Ceramic Materials | | | | | |
|---|---|---|---|---|---|
| Materials | Softening Temperature °C | Coefficient Thermal Exp. 30-400 10X-6/°C | Fracture Toughness KLMPa m^{0.5} | True Harness H°GPa | Young's Modulus GPa |
| NAB/CG/7c | 975 | 2.03±0.09 | 1.78±0.02 | 5.57±0.36 | 116.19±0.42 |
| NAB/CG/10a | 863 | 3.90±0.05 | 1.60±0.02 | 4.45±0.20 | 96.11±0.2 |
| NAB/CG/10b | 863 | 4.95±0.09 | 1.44±0.02 | 4.66±0.35 | 112.09±0.83 |
| NAB/CG/L1 | 1063 | 4.72±0.33 | 1.30±0.02 | 4.86±0.07 | 90.89±0.69 |
| NAB/CG/L3 | 1025 | 5.38±0.28 | 1.35±0.01 | 5.14±0.10 | 96.18±0.25 |
| NAB/CG/L4a | 1013 | 5.88±0.15 | 1.13±0.02 | 5.09±0.08 | 111.48±0.57 |
| NAB/CG/L5 | 1075 | 5.83±0.10 | 1.44±0.02 | 5.78±0.90 | 113.08±0.41 |
| Dicor™(as cast) | | | 1.12±0.01 | | 63.79 |
| Dicor™(Cerammed) | | | 1.22±0.02 | | 64.63 |

It will therefore, be appreciated that a dental construct according to the present invention accomplishes the objects of the invention as set forth above, and otherwise constitutes a novel. unique and heretofore unknown contribution to the art.

## Claims

1. A non-metallic dental construct comprising a glass ceramic coping having a resin composite material in the form of a veneer affixed to at least a portion of the coping.

2. The non-metallic construct as in claim 1, wherein said coping comprises a material selected from the group consisting of K, Mg, Si, Li, P, La, Ce, Y, Ba, Pb, Ta, Ca, Na, and mixtures thereof.

3. The non-metallic construct as in claim 2, wherein the coping material comprises an oxide or a fluoride.

4. The non-metallic dental construct as in claim 3 wherein said coping material is selected from a group consisting of K₂O-MgF₂-MgO-SiO₂; Li₂O-CaO-Al₂O₃-SiO₂-X, Al(PO₃)₃-SiO₂-Li₂O-CaX and Li₂-ZnO-P₂O₅-X, where X is a network modifier consisting from a group consisting of TiO₂, ZrO₂, La₂O₃, CeO₂, Y₂O₃, ZnO, MgO, BaO, PbO, Ta₂O₅, Li₂O, K₂O, CaF₂, AlF₃, BaF₂ and Na₂O.

5. The non-metallic dental construct as in claim 1 wherein the resin composite material is a dimethacrylate resin or a urethane.

6. The non-metallic dental construct as in claim 5 wherein the resin composite material is blended with a finely divided filler material.

7. The non-metallic dental construct as in claim 6 wherein the filler material is selected from a group consisting of calcium aluminosilicate, zirconium silicate, barium or strontium aluminosilicate glass or ceramics.

8. The non-metallic dental construct as in claim 7 wherein said filler material has a particle size of from 0.04 to 10 microns.

9. The non-metallic dental construct as in claim 7 wherein the filler material is surface treated to provide adhesion to the resin matrix and said glass or ceramic filler material.

10. The non-metallic dental construct as in claim 9 wherein the filler material is surface treated using a silane material.

## Patentansprüche

1. Nichtmetallisches Dentalkonstrukt, das eine Glas-Keramik-Abdeckung aufweist, die einen Harz-Kompositwerkstoff in Form eines auf mindestens einem Teil der Abdeckung fixierten Überzugs besitzt.

2. Nichtmetallisches Konstrukt nach Anspruch 1, worin die Abdeckung ein Material aufweist, ausgewählt aus der Gruppe bestehend aus K, Mg, Si, Li, P, La, Ce, Y, Ba, Pb, Ta, Ca, Na und Mischungen davon.

3. Nichtmetallisches Konstrukt nach Anspruch 2, worin das Abdeckungsmaterial ein Oxid oder ein Fluorid aufweist.

4. Nichtmetallisches Dentalkonstrukt nach Anspruch 3, worin das Abdeckungsmaterial ausgewählt ist aus der Gruppe bestehend aus K₂O-MgF₂-MgO-SiO₂; Li₂O-CaO-Al₂O₃-SiO₂-X, Al(PO₃)₃-SiO₂-Li₂O-CaX und Li₂-ZnO-P₂O₅-X, worin X ein Netzwerk-Modifier ist, ausgewählt aus einer Gruppe bestehend aus TiO₂, ZrO₂, La₂O₃, CeO₂, Y₂O₃, ZnO, MgO, BaO, PbO, Ta₂O₅, Li₂O, K₂O, CaF₂, AIF₃, BaF₂ und Na₂O.

5. Nichtmetallisches Dentalkonstrukt nach Anspruch 1, worin der Harz-Kompositwerkstoff ein Dimethacrylatharz oder ein Urethan ist.

6. Nichtmetallisches Dentalkonstrukt nach Anspruch 5, worin der Harz-Kompositwerkstoff mit einem fein zerteilten Füllstoffmaterial gemischt ist.

7. Nichtmetallisches Dentalkonstrukt nach Anspruch 6, worin das Füllstoffmaterial ausgewählt ist aus einem/einer Calciumaluminium-, Zirkoniumsilikat-, Barium- oder Strontiumaluminiumsilikat-Glas oder -Keramik.

8. Nichtmetallisches Dentalkonstrukt nach Anspruch 7, worin das Füllstoffmaterial eine Teilchengröße von 0,04 bis 10 µm aufweist.

9. Nichtmetallisches Dentalkonstrukt nach Anspruch 7, worin das Füllstoffmaterial oberflächenbehandelt ist, um eine Adhäsion zwischen Harz-Matrix und Glas- oder Keramik-Füllstoffmaterial zu ergeben.

10. Nichtmetallisches Dentalkonstrukt nach Anspruch 9, worin das Füllstoffmaterial unter Verwendung eines Silanmaterials oberflächenbehandelt ist.

## Revendications

1. Assemblage dentaire non métallique comprenant une chape vitrocéramique comportant un matériau composite de résine sous forme d'un revêtement fixé à au moins une partie de la chape.

2. Assemblage non métallique suivant la revendication 1, dans lequel ladite chape comprend une matière choisie dans le groupe consistant en K, Mg, Si, Li, P, La, Ce, Y, Ba, Pb, Ta, Ca, Na et leurs mélanges.

3. Assemblage non métallique suivant la revendication 2, dans lequel la matière de chape comprend un oxyde ou un fluorure.

4. Assemblage dentaire non métallique suivant la revendication 3, dans lequel ladite matière de chape est choisie dans les groupes consistant en K₂O-MgF₂-MgO-SiO₂; Li₂O-CaO-Al₂O₃-SiO₂-X, Al(PO₃)₃-SiO₂-Li₂O-CaX et Li₂-ZnO-P₂O₅-X, dans lesquels X représente un modificateur de réseau consistant en modificateur choisi dans le groupe consistant en TiO₂, ZrO₂, La₂O₃, CeO₂, Y₂O₃, ZnO, MgO, BaO, PbC, Ta₂O₅, Li₂O, K₂O, CaF₂, AlF₃, BaF₂ et Na₂O.

5. Assemblage dentaire non métallique suivant la revendication 1, dans lequel le matériau composite de résine est une résine de diméthacrylate ou un uréthanne.

6. Assemblage dentaire non métallique suivant la revendication 5, dans lequel le matériau composite de résine est mélangé à une matière de charge finement divisée.

7. Assemblage dentaire non métallique suivant la revendication 6, dans lequel la matière de charge est choisie dans le groupe consistant en aluminosilicate de calcium, silicate de zirconium, verre d'aluminosilicate de baryum ou de strontium et matières céramiques.

8. Assemblage dentaire non métallique suivant la revendication 7, dans lequel ladite matière de charge a un diamètre de particule de 0,04 à 10 micromètres.

9. Assemblage dentaire non métallique suivant la revendication 7, dans lequel la matière de charge est traitée en surface pour assurer l'adhérence à la matrice de résine et ladite matière de charge consistant en verre ou matière céramique.

10. Assemblage dentaire non métallique suivant la revendication 9, dans lequel la matière de charge est traitée en surface en utilisant une matière du type silane.
